# EUROPEAN PATENT SPECIFICATION

(11) **EP 2 708 643 B1**
(45) Date of publication and mention of the grant of the patent: **08.11.2017**
(21) Application number: 12425153.9
(22) Date of filing: 14.09.2012
(51) Int. Cl.: B07B 1/00, B07B 15/00, B02C 23/14, C12P 3/00, C12P 7/10, C12P 7/16, D21B 1/06

(54) **Method for pretreating biomasses prior to conversion to biofuel**
Verfahren zur Vorbehandlung von Biomasse als Vorstufe zur Konvertierung zu Biokraftstoff
Procédé de prétraitement de biomasses avant la conversion au biocarburant

(43) Date of publication of application: 19.03.2014
(73) Proprietor: Foodoverseas S.r.l., 00131 Roma (IT)
(72) Inventor: La Froscia, Sabatino, 85040 Viggianello (Potenza) (IT)
(74) Representative: Bianciardi, Ezio

(56) References cited:
- DE-A1-102011 012 191
- US-A- 2 452 533
- US-A- 4 562 969
- US-A1- 2005 136 520

## Description

This invention relates to a method for pretreating biomasses to obtain biofuel.

The invention thus addresses the technical field of biofuels.

In the biofuels sector it is known that biofuel can be obtained from biomasses, meaning by the term "biomasses" any matter of vegetable or animal origin and the organic fraction of solid urban wastes.

The term "biofuel" is used to mean any liquid or gaseous fuel derived from biomasses such as, for example, biogas, biomethane, biohydrogen, bioethanol, biobutanol.

In order to produce biofuels, the polysaccharides making up the biomasses must undergo hydrolysis, that is to say, they must be broken down into simpler sugars, monosaccharides or oligosaccharides.

The simpler sugars constitute the nutritional substrate of the microorganisms - bacteria, yeasts or fungi - which allow the biomass to undergo biochemical conversion to biofuel. The term "biochemical conversion", or "fermentation", or "digestion" denotes the conversion of one molecule to another, catalyzed by microorganisms and/or enzymes.

One disadvantage of biofuel production processes known up to now is the reduced hydrolytic efficiency of a wide variety of biomasses, especially cellulosic biomasses, meaning by "cellulosic biomasses" those biomasses which contain cellulose, often associated with other polysaccharides -hemicelluloses- and with a phenolic polymer called lignin. Examples of cellulosic biomasses are wood, cereal straw, herbaceous plants, cow manure, the by-products of cereal milling, etc.

The reduced hydrolytic efficiency is caused by the lignin, which reduces enzyme action on the hydrolyzable carbohydrates, and by the crystal structure of the cellulose, which creates an obstacle to enzyme action.

The problem constituted by the reduced efficiency of the hydrolysis process has greatly limited production, on a large scale, of biofuels not derived from cereals, sugar cane or oilseeds.

In order to overcome this disadvantage, several methods have been proposed for the pretreatment of the biomasses before subjecting the biomasses to the hydrolysis treatment.

These pretreatment methods make it possible to partly remove the lignin, reduce the crystallinity of the cellulose, facilitating its depolymerization and thereby improving hydrolysis efficiency, in order to enable the biomasses to be efficiently and economically converted to methane, ethanol or other biofuels.

Prior art pretreatment methods can be divided into chemical, physical and biological pretreatment methods and combinations thereof.

Pretreatment in an acid environment is a very common chemical pretreatment method. Its main disadvantage is the formation of microbial growth inhibitors which reduce the efficiency of the process of converting the biomasses to biofuel. Moreover, processes of chemical pretreatment with sulphuric acid cannot be used to promote anaerobic degradation of the biomasses into biogas because the gas produced is polluted.

In any case, since the acid solutions are highly aggressive, treatment must be carried out in structures made of special steel or specific non-metallic materials, all very expensive.

To overcome these difficulties, chemical pretreatment processes in an alkaline environment have been developed. These produce effluents which must be treated and involve high energy consumption and are therefore unsuitable for industrial use. Biomass pretreatment methods of a physical type, such as sonication and irradiation with microwaves and ionizing rays, have also been developed. Examples of these processes are described in US2005/0136520, WO2009/134745 and US2012/0111322.

It is also know from DE 102011012191 a method for pretreating biomasses in order to obtain fibers of cellulose for the production of paper.

The method of this document uses pulpers and ball mills which require very high power consumption necessary for the pretreatment of biomasses intended for producing biofuel.

Document US 2452533 discloses the treatment of straw to produce a type of fiber suitable for the production of paper or other products but not biofuels. The method uses percussive grinding by means of ball mills and rod mills which require high power consumption.

Document US 4562969 relates to a method for preparing groundwood pulp for the paper industry obtained by grinding wood in water, in particular for the production of paper for newspapers and/or soft crepe paper which require high power consumption.

These processes are, however, particularly expensive and difficult to apply at industrial level.

In all the pretreatment methods described, the milling and/or sorting of the biomass is used only to prepare the biomass for pretreatment in order to facilitate the action of the chemical agents, heat, radiation, etc.

The aim of this invention is to provide a biomass pretreatment method which can guarantee a high yield of biomass conversion to biofuels and an improvement in the quality of the biofuels themselves.

A further aim of the invention is to provide a biomass pretreatment method with reduced energy consumption.

A yet further aim of the invention is to provide a biomass pretreatment method which does not produce potentially polluted effluents.

According to the invention, these aims are achieved by a biomass pretreatment method comprising the technical features set out in one or more of the accompanying claims.

The technical features of the invention, according to the above aims, are clearly described in the appended claims and its advantages are apparent from the detailed description which follows, with reference to the accompanying drawings which illustrate preferred non-limiting example embodiments of it, and in which
- Figures 1 to 6 illustrate respective embodiments of the method according to the invention;
- Figure 7 shows a graph representing the quantities of methane obtainable by subjecting the biomasses to pretreatment with the method of the invention, compared to prior art pretreatment methods;
- Figure 8 is a graph showing the ratio between the energy used for pretreatment and the energy generated by burning the methane produced, comparing the method of the invention with some prior art pretreatment methods.

The biomass pretreatment method according to the invention allows the biomasses to be pretreated so that a biofuel can subsequently be obtained using known processes (in particular, biochemical conversion).

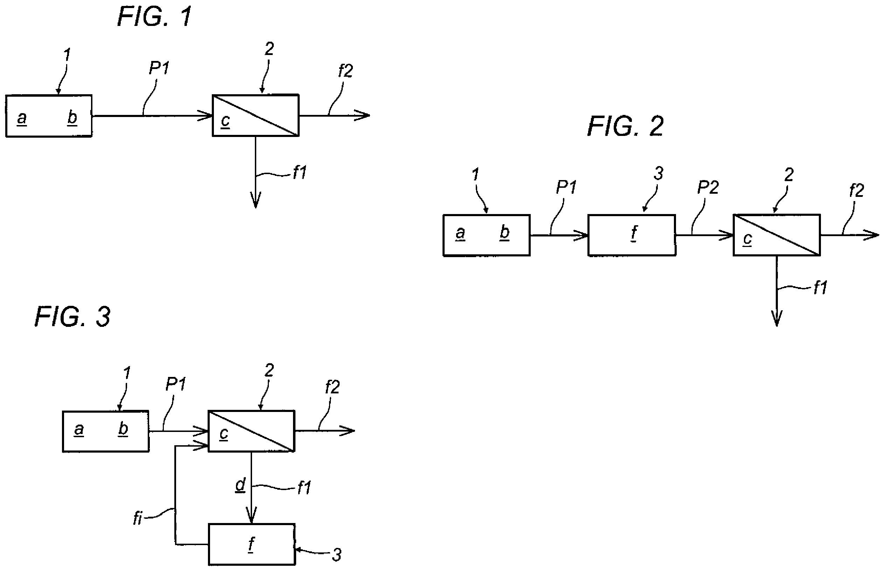

With reference to Figure 1, which schematically illustrates a simplified form of the biomass pretreatment method of the invention, the method comprises an initial step a) of causing the biomasses to pass through a first size reduction device 1 to enable the biomasses to be reduced to smaller-sized particles.

The first size reduction device 1 is a knife mill or a hammer mill.

It should be noted that the knife or hammer mill 1 advantageously has a lower energy consumption than other particle size reduction devices if it is used to reduce the size of large size particles (where "large" means greater than 5 mm).

It should be noted, therefore, that this type of mill 1 is particularly effective for the pretreatment of coarse size particles and thus allows such particles to be reduced in size in energy efficient manner.

It should be noted, as will become clearer below, where the various different steps are described in more detail, that in the method of the invention each step is performed by a predetermined type of mechanical treatment device associated with optimum energy consumption for that step.

Thus, the method according to the invention is, as a whole, more energy efficient.

The method of the invention comprises a further step b) of placing the ground particles on a particle filtering grille so that particles P1 smaller than a predetermined size pass through the grille and feed out of the size reduction device 1. Thus, it should be noted that the grinding mill 1 mechanically treats in an energy efficient manner particles which are larger than the predetermined size.

The filtering grille is preferably built into the grinding mill 1.

The filtering grille is configured in size such as to allow particles smaller than the predetermined size to pass through it.

Preferably, the predetermined size is 5 mm: according to this aspect, the filtering grille allows particles P1 smaller than 5 mm in diameter to pass through it.

The particles P1 which pass through the filtering grille are fed out of the grinding mill 1 towards a further processing step c).

The next processing step c) comprises making the particles P1 pass through a first particle classifying device 2 to allow the particles to be divided according to their size into a first fraction f1 of coarse size particles and a second fraction f2 of fine size particles.

It should be noted therefore that the first fraction f1 of coarse size particles includes larger size particles, whilst the second fraction f2 of fine size particles includes particles which are smaller in size than those of the first fraction f1 of coarse size particles.

The particles f1 of coarse size are larger than 600 µm in size, whilst those of fine size f2 are smaller than 600 µm in size.

The classifying device 2 consists of a centrifugal, air stream classifier (known as "vortex air classifier") or a centrifugal sieve.

Figure 2 illustrates another embodiment of the method of the invention where after the step b) of passing the particles through the grille and before passing the particles P1 (which are smaller than the predetermined size) through the particle classifying device 2, there is a further step f) of passing the particles P1 which are smaller than the predetermined size through a second mechanical size reduction device 3, to obtain finer particles P2.

It should be noted therefore that the particles denoted P2 in Figure 2 are smaller in size than those denoted P1 in Figure 2.

Preferably, the second mechanical size reduction device 3 integrates the first classifying device 2.

Figure 3 illustrates a yet further embodiment of the method of the invention where the step f) of passing the particles P1 which are smaller than the predetermined size through the second mechanical size reduction device 3 is performed after the particles P1 have been fed into the first particle classifying device 2.

In practice, the method schematically represented in Figure 3 integrates that of Figure 1 and according to it, the fraction f1 of particles of coarse size fed out of the first classifying device 2 are passed through the second size reduction device 3 to obtain a fraction fi of finer size. Thus, more generally speaking, in the treatment method illustrated in Figures 2 and 3, the step b) of passing the particles through the grille is followed by the step f) of passing the particles P1 which are smaller than the predetermined size through a second mechanical size reduction device 3, in order to obtain particles (P2,fi) of still finer size.

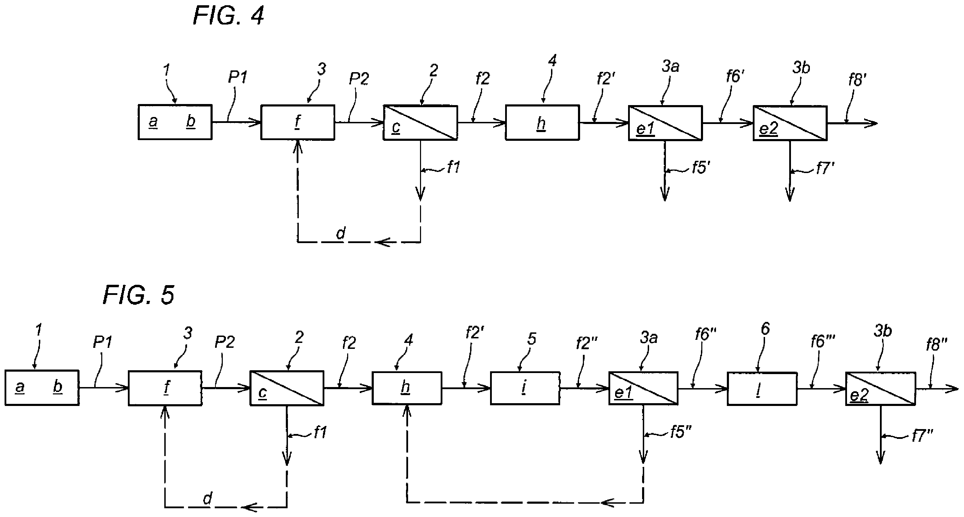

With reference to the diagrams of Figures 4, 5 and 6, the second mechanical size reduction device 3 is upstream of the first classifying device 2 and the fraction f1 of coarse size particles fed out of the first classifying device 2 is fed back to the second mechanical size reduction device 3. According to this aspect, the fraction f1 of coarse size particles mixes with the particles P1 fed out of the mill 1 in the second mechanical size reduction device 3, to undergo further size reduction.

Preferably, the mechanical treatment device 3 may be a roller mill or, still more preferably, a centrifugal impact mill.

It should be noted that the centrifugal impact mill is more energy efficient than the knife or hammer mill 1 in reducing the size of smaller particles: thus, in the method according to the invention, the centrifugal impact mill treats the particles mechanically after the knife or hammer mill 1. Preferably, the pretreatment method further comprises one or more steps (e1,e2,e3) of making the fraction f2 of fine size particles pass through one or more further particle classifying devices (3a,3b,3c) to allow the fraction f2 of fine size particles to be divided into two or more superfine fractions (f5,f7,f9,f10) of different fineness (Figures 4, 5, 6), where "superfine" means fractions with grain size smaller than that of the fraction f2.

Each superfine fraction (f5,f7,f9,f10) has particles of predetermined size.

Preferably, the superfine fractions (f5,f7,f9,f10) have the following sizes (expressed in terms of D50, that is to say, the size of the filtering device which 50% of the material passes through): 350 µm, 200 µm, 90 µm and less than 90 µm.

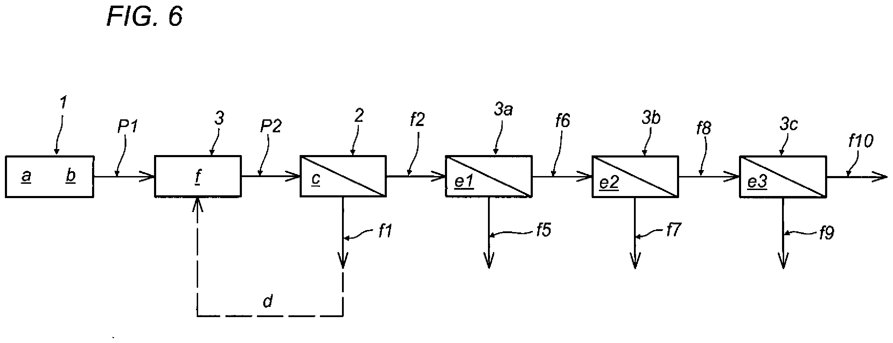

In this regard, Figure 6 shows a plurality of classifying devices (3a,3b,3c).

More in detail, downstream of the first classifying device 3a, there are a second classifying device 3b and a third classifying device 3c connected to each other in cascade, that is, one after the other.

The second classifying device 3a receives the fraction f2 of fine size particles and divides it into a fraction f5 of particles of superfine size and into a fraction f6 still finer than the fraction f5.

The third classifying device 3b receives the fraction f6 from the classifying device 3a and divides it into a fraction f7 of particles finer than the fraction f6 and into a fraction f8 still finer than the fraction f7.

The fourth classifying device 3c receives the fraction f8 from the classifying device 3b and divides it into a fraction f9 of particles finer than the fraction f8 and into a fraction f10 still finer than the fraction f9.

Preferably, the fraction f10, which is in suspension in an air stream in the fourth classifying device 3c, is separated from the air stream by making it pass through a separation device.

The separation device may be a separating cyclone.

It should also be noted that the carrier fluid may be air, or an inert gas such as, for example, CO₂, or N₂, or a non-inert gas such as, for example, NH₃ .

It should be noted that inert gases allow advantageously to prevent the formation of explosive mixtures.

The classifying devices (3a,3b,3c) allow a particularly precise and diversified subdivision of biomass particle size to be obtained.

With reference to Figures 4 and 5, the fine size particles f2 are made to pass through a third size reduction device 4 before being fed to the second classifying device 3a.

With reference in particular to Figure 4, the step h) is followed by a step of feeding the particles f2' into the second classifying device 3a to obtain fine fractions f5' and finer fractions f6'.

This step is in turn followed by a step of feeding the particles f6' into the third classifying device 3b to obtain finer and finer fractions f7' and f8'. In the diagram shown in Figure 5, the particles f2 pass alternately through one or more size reduction devices (5,6) and one or more classifying devices (3a,3b) to obtain a plurality of fine fractions (f5'',f7'',f8'') of different fineness.

With reference in particular to Figure 5, it may be observed that after the step h) there is a step i) of making the particles f2' leaving the size reduction device 4 pass through a fourth size reduction device 5, to obtain still finer particles f2".

After the step i), the particles f2" are fed to the second classifying device 3a in order to divide them into a superfine fraction f5" and into another fraction f6" of still finer size.

More specifically, according to the embodiment of Figure 5, the superfine fraction f5" is fed back to the third size reduction device 4 where it undergoes further size reduction.

It should be noted that the fraction f6" is made to pass through a fifth size reduction device 6 and then through the third classifying device 3b to obtain other superfine fractions f7" and f8". Preferably, the third mechanical treatment device 4 is a centrifugal impact mill or, alternatively, a ball mill or a vibro energy mill.

Preferably, the fourth mechanical treatment device 5 is a jet mill or, alternatively, a ball mill or a vibro energy mill.

It should also be noted that if the fourth size reduction device 5 and/or the fifth size reduction device 6 is a jet mill, it may integrate the classifying device (3a, 3b) - which is located downstream of the selfsame mechanical treatment device (5,6) - so as to define a single size reduction and classifying device.

The particles which are fed into the jet mill collide with the particles already inside it, thereby reducing the size of the particles.

Thus, size reduction in the jet mill occurs by collision of the particles with each other, a particularly effective mechanism in the case of particles with a superfine grain size.

All the embodiments of Figures 4, 5 and 6 can also be made by producing the fine material f2 according to the embodiment of Figure 3.

The availability of a plurality of particle fractions with a grain size from medium (f1), to fine and superfine (f5,f5',f5",f7,f7',f7",f8,f8', f8'',f9,f10) means that each fraction can be used for a specific purpose as a function of its size and chemical composition.

For example, the coarser fractions (f5) can be used to produce biogas in a process of anaerobic digestion, those of intermediate size (f7,f8), which are highly appreciated for making biofuel, can be used in a process of fermentation to produce ethanol and the superfine fractions (f9,f10), which are still more appreciated, can be fermented to produce hydrogen.

The superfine fractions can also be used in biochemical processes to produce organic acids such as, for example, acetic acid , levulinic acid and lactic acid, which are platform molecules (or "building blocks") used to obtain high value chemicals by synthesis.

Again by way of example, the less fine fractions (f2,f5) are generally higher in fibre content and lower in ash content, which means that, besides fuel production, they can also alternatively be used for the production of fuel pellets and for the cellulose industry.

The term "fuel pellets" is used to mean a solid fuel obtained by densifying a biomass which has been reduced in size and which is then compressed into cylindrical capsules normally between 6 and 10 mm in diameter.

The pellets are normally used to feed small- and medium-sized boilers.

Superfine fractions obtained from some biomasses such as, for example, residues from the processing of oranges, pineapples, olives and grapes, can also, advantageously, be used for the production of food additives and nutraceutical products.

Again by way of example, the superfine fractions (for example, f9,f10) which are particularly rich in non-structural carbohydrates and proteins can be used as animal feeds (in the form of meal).

The method described above makes it possible to obtain particularly advantageous results.

In particular, thanks to the method of pretreatment prior to hydrolysis, it is possible to obtain high-quality biofuel, with a low content of undesirable substances (for example, hydrogen sulphide).

It should also be noted that the pretreatment process described gives biofuel with a particularly high energy yield: in other words, the energy used in the mechanical pretreatment and in the subsequent process - for example, anaerobic fermentation - is considerably less than the energy obtainable from the biofuel made.

It should be noted that size reduction of the biomasses facilitates the convertibility of the biomasses into biofuel, as explained in more detail below.

It should also be noted that the higher content of non-structural carbohydrates obtained with the method of the invention by depolymerization of cellulose and hemicellulose, also facilitates the convertibility of the biomasses into biofuel.

The invention also defines a method for the production of biofuel comprising the pretreatment method described above, followed by a step of biochemical conversion of the treated particles in order to obtain biofuel.

The example which follows, derived from experimental tests, highlights the advantages of the pretreatment method according to the invention. The tables below report data regarding cereal straw with 12% humidity.

The cereal straw has been processed using the method described above, where:
- the device 1 is a hammer mill with a grille of 5 mm mesh size;
- the device 3 is a centrifugal impact mill.

The method allowed three fractions to be obtained, namely C (corresponding to f1), M (corresponding to f5 or f5' or f5") and F (corresponding to f6 or f6' or f6"), which constitute 40.4%, 45.1% and 14.5% of the raw material input, respectively. Fraction C was found to have a D50 (characteristic mesh size of a filter through which 50% of the material passes) of 600 µm, fraction M a D50 of 350 µm and fraction F a D50 of 200 µm.

Thus, fraction C has the largest grain size, fraction F, the finest grain size and fraction M, the intermediate grain size.

The table below shows the chemical composition of the raw cereal straw used and the fractions C,M,F obtained using the method of the invention.

| | **Ash (% TS)** | **NDF (% VS)** | **ADF (% VS)** | **ADL (% VS)** | **CP (% VS)** | **EE (% VS)** | **NSC (% VS)** |
|---|---|---|---|---|---|---|---|
| **raw cereal straw** | **8.6** | **84.2** | **51.7** | **8.6** | **8.0** | **1.5** | **6.3** |
| **C** | **7.1** | **83.7** | **54.9** | **8.8** | **6.9** | **0.9** | **8.4** |
| **M** | **8.5** | **60.3** | **52.7** | **9.2** | **8.2** | **1.4** | **10.0** |
| **F** | **11.8** | **69.8** | **42.0** | **6.8** | **11.5** | **2.2** | **16.5** |

The following notation is used in the table above: Ash is the ash content;
NDF is the neutral detergent fibre content;
ADF is the acid detergent fibre content;
ADL is the acid detergent lignin content;
CP is the crude protein content;
EE is the ether extract content (lipids);
NSC is the non-structural carbohydrate content;
TS is the total solid content;
VS is the volatile solid content (otherwise known as organic substance).

The method of the invention can produce a change in the chemical composition of the material, although it is a mechanical method. This chemical modification of the material may be inferred from the above table (which shows the chemical composition of the material subjected to the process and of the fractions C,M,F obtained from the process according to the invention).

In effect, as shown in the table, the non-structural carbohydrate (NSC) content of the fractions C, M, F is greater than that of the raw material and the total fibre (NDF) content is always less than that of the raw material.

That means the method of the invention advantageously allows the complex carbohydrates forming part of the fibre (cellulose and hemicellulose) to be converted to simpler carbohydrates which are easier to hydrolyze. Moreover, the data in the table related to the fractions C, F, M, in comparaison with the raw material, show the differences in terms of chemical composition between the material produced and the raw material.

The fraction F, for example, is 2.6 times higher in non-structural carbohydrate (NSC) content than the raw material and 1.4 times higher in crude protein (CP) and ether extract (EE) content than the raw material.

The fraction F also has a lower fibre (NDF) content than the raw material (69.8% for the fraction F as against 84.2% for the raw material).

The products, or fractions, obtained with the method according to the invention were subjected to a biomethanation test under mesophilic conditions (that is, at temperatures in the range of 30-45°C) for a period of 52 days, clearly showing a higher methane producing capacity compared to untreated material ("raw cereal straw"), as may be inferred from the table below.

| | **CH₄ yield (Nm³/tᵥₛ)** | **CH₂ (% vol)** | **NH₃ (ppm)** | **H₂S (ppm)** |
|---|---|---|---|---|
| **raw cereal straw (3 cm)** | **152** | **52,1** | **36** | **63** |
| **C** | **298** | **51.1** | **35** | **46** |
| **M** | **319** | **52.3** | **33** | **51** |
| **F** | **354** | **52.2** | **35** | **57** |

Another advantage of the invention is that it improves the quality of the fuel produced, as may be inferred from the reduced hydrogen sulphide content in the biogas produced with the fractions C, M and F obtained with the method of the invention compared to the biogas obtained from untreated straw.

The following notation is used in the table above: CH₄ yield is the methane output (in normal cubic metres per tonne of organic substance, where "normal cubic metres" means the volume of the methane gas m³ measured at reference conditions of 1 atm and temperature of 20°C);
CH₄ is the methane content in the biogas;
NH₃ is the ammonia content in the biogas;
H₂S is the content of hydrogen sulphide - an undesirable substance - in the biogas.

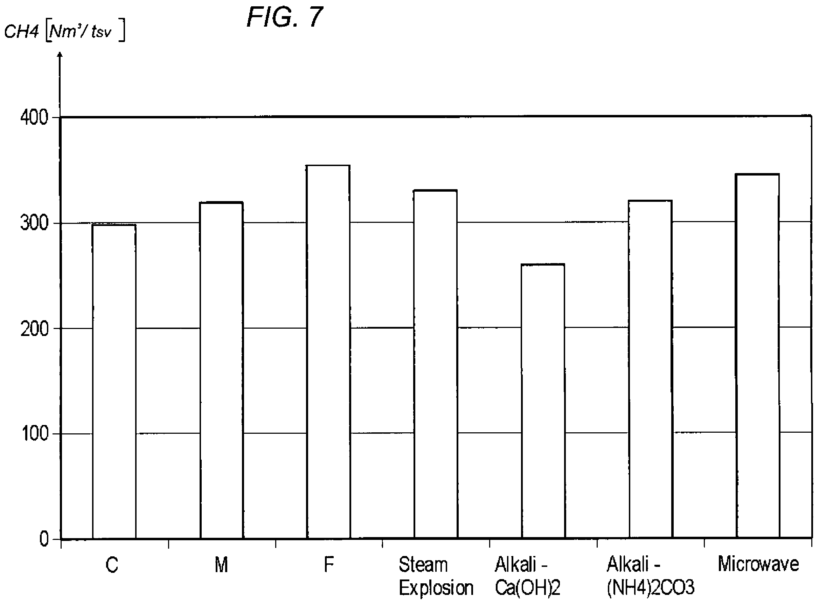

Figure 7 graphically compares the methane output from each of the fractions C, M, F obtained with the method of the invention (followed by anaerobic digestion under mesophilic conditions) and from products obtained from known methods such as steam explosion, chemical with alkali Ca(OH)₂, chemical with alkali (NH₄)₂CO₃, and microwave irradiation.

The results obtained with the pretreatment method of the invention are better than those obtained with prior art pretreatment methods which are more complex and expensive, as illustrated in Figure 7. In particular as regards the very fine fraction F, the biogas output is a definite improvement over that that from prior art processes.

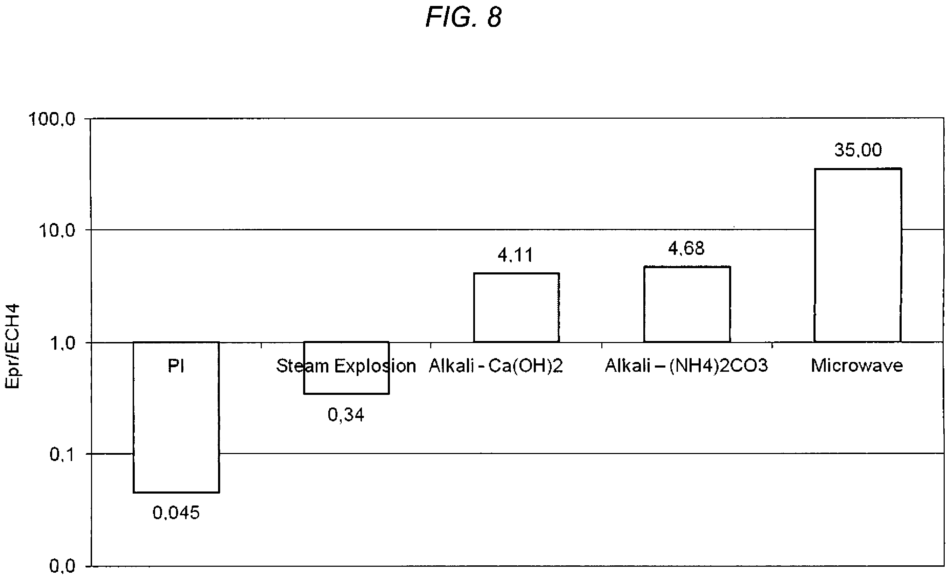

Figure 8 graphically compares the ratio between the energy used for the pretreatment (Epr) and that obtained by burning the methane (ECH4) produced from the subsequent biochemical conversion (in this specific example, the conversion is achieved by anaerobic digestion), for the method according to the invention and prior art methods.

If the Epr/ECH4 ratio is greater than 1, the energy used for pretreatment is greater than that obtainable by burning the methane produced, and the process thus has a negative energy balance.

By way of example, the Epr/ECH4 ratio is 4.1 in chemical pretreatment with alkali Ca(OH)₂: thus, pretreating the material chemically with alkali Ca(OH)₂ uses more than 4 times the energy that will be obtainable with the methane produced. The prior art methods compared are steam explosion, chemical with alkali Ca(OH)₂, chemical with alkali (NH₄)₂CO₃, and microwave irradiation.

The following notation is used in Figure 8:
ECH4 is the energy generated by burning the methane produced;
Epr is the energy used for the pretreatment (electrical energy was equalized with the thermal energy generated by burning the methane produced, considering a production and distribution yield of 0.35);
PI is the method according to this invention.

The results obtained with the pretreatment method of the invention are clearly better than those obtained with prior art pretreatment methods. Below are some details regarding calculation of the Epr/ECH4 ratio to obtain the graph of Figure 8. With reference to the steam explosion process, the data necessary for calculating the Epr/ECH4 ratio were derived from the description of the process in the technical literature, in particular A. Bauer et al., Analysis of methane potentials of steam-exploded wheat straw and estimation of energy yields of combined ethanol and methane production, Journal of Biotechnology 142 (2009) 50-55.

With reference to the chemical processes with alkali Ca(OH)₂" and alkali (NH₄)₂CO₃, the data necessary for calculating the Epr/ECH4 ratio were derived from the description of the treatment in the technical literature: TV Fernandes et al., Effects of thermo-chemical pre-treatment on anaerobic biodegradability and hydrolysis of lignocellulosic biomass, Bioresource Technology, 100 (2009), 2575-2579).

With reference to the microwave irradiation process, the data necessary for calculating the Epr/ECH4 ratio were derived from the description of the treatment in: D. Jackowiak et al., Optimisation of a microwave pretreatment of wheat straw for methane production, - Bioresource technology, 102 (2011) 6750-6756).

The energy requirement of the pretreatment of this invention is at least one order of size less than that of known pretreatment methods, as shown in Figure 8.

Another advantage of the invention is that it does not produce waste products (such as liquid effluents).

The invention described above is susceptible of industrial application and may be modified and adapted in several ways without thereby departing from the scope of the inventive concept. The scope of the invention is defined by the claims only.

## Claims

1. A method for pretreating biomasses to allow their subsequent conversion to biofuel, **characterized in that** it comprises the following steps:
- a) causing the biomasses to pass through a first mechanical size reduction device (1) formed by a knife mill or a hammer mill to enable the biomasses to be reduced to particles;
- b) placing the particles on a particle filtering grille so that particles (P1) smaller than a predetermined size of 5mm pass through the grille and feed out of the first mechanical size reduction device (1);
- c) making the particles (P1) which are smaller than the predetermined size pass through a first particle classifying device (2) to allow the particles (P1) which are smaller than the predetermined size to be divided according to size into a fraction (f1) of coarse size particles and a fraction (f2) of fine size particles, wherein in the fraction (f1) of coarse size particles said particles are larger than 600 µm in size, whilst in the fraction (f2) of fine size particles said particles are smaller than 600 µm in size, said first classifying device (2) being a vortex air classifier or a centrifugal sieve.

2. The method according to claim 1, wherein the step b) of passing the particles through the grille is followed by a step f) of passing the particles (P1) which are smaller than the predetermined size and are fed out from the first mechanical size reduction device (1), or from the grille, through at least one second mechanical size reduction device (3), to obtain particles (P2,fi) which are smaller in size than the particles (P1) fed out of the first mechanical size reduction device (1).

3. The method according to claim 2, wherein the step f) of passing the particles (P1) which are smaller than the predetermined size through the second mechanical size reduction device (3) is carried out before feeding the particles (P1) which are smaller than the predetermined size into the first particle classifying device (2).

4. The method according to any of the preceding claims 2 or 3, comprising a step of passing the fraction (f1) of particles of coarse size fed out of the first classifying device (2) through the second mechanical size reduction device (3) to obtain a fraction (fi) of finer size.

5. The method according to claim 4, comprising a step of passing the particles of finer size (fi) through the first classifying device where they mix with the particles (P1) fed out of the first mechanical size reduction device (1).

6. The method according to any of the preceding claims, comprising at least one step (el,e2,e3) of making the fraction (f2) of fine size particles pass through at least one second particle classifying device (3a,3b,3c) to allow the fraction (f2) of fine size particles to be dimensionally divided into two or more superfine fractions (f5,f5',f5'',f7,f7',f7'', f8,f8',f8",f9,f10), each having particles of a predetermined superfine size.

7. The method according to any of the preceding claims 2-5, comprising at least one step (h) of making the fraction (f2) of fine size particles pass through at least one third mechanical particle size reduction device (4) in order to carry out a third reduction of the size of the particles of the fraction (f2).

8. The method according to claim 7, wherein the at least one step (h) precedes the at least one step (e1) of making the fraction (f2) of fine size particles pass through the second particle classifying device (3a, 3b, 3c).

9. The method according to any of the preceding claims 2-5, 7 or 8, comprising at least one step (i,e1;l,e2) of making the fraction (f2) of fine size particles pass through at least one fourth size reduction device (5,6) and at least one subsequent third particle classifying device (3b,3c) to allow the size of the particles to be gradually reduced and to allow the fraction (f2) of fine size particles to be dimensionally divided into two or more superfine fractions (f5", f7",f8").

10. The method according to any of the preceding claims 6 or 9, wherein in the superfine (f5,f5',f5",f7,f7',f7", f8,f8',f8",f9,f10) fractions of particles of predetermined superfine size said particles are approximately the following size, respectively: 350 µm, 200 µm, 90 µm and less than 90 µm.

11. The method according to any of the preceding claims 6, 8 or 9, wherein the further classifying devices (3a,3b,3c) are vortex air classifiers or centrifugal sieves.

12. The method according to any of the preceding claims 2-5 or 7-9, wherein the second mechanical size reduction device (3) is a centrifugal impact mill.

13. The method according to any of the preceding claims 2-5, 7-9 or 12, wherein the second mechanical size reduction device (3) is built into the first classifying device (2), or vice versa.

14. The method according to any of the preceding 2-5, 7-9, 12 or 13, wherein the second mechanical size reduction device (3) is a grinding device or roller mill.

15. A method for obtaining biofuel from biomasses, comprising the steps of:
- treating the biomasses using the pretreatment method according to any of the preceding claims;
- subjecting the particles obtained from the pretreatment method according to the preceding claims to biochemical conversion in order to obtain biofuel.

## Patentansprüche

1. Verfahren zur Vorbehandlung von Biomassen, um deren nachträgliche Konvertierung zu Biokraftstoffen zu ermöglichen, **dadurch gekennzeichnet, dass** es die folgenden Schritte umfasst:
- a) Veranlassen, dass die Biomasse durch eine erste mechanische Grössenreduktionsvorrichtung (1), die durch eine Messermühle oder eine Hammermühle gebildet wird, durchläuft, um zu ermöglichen, dass die Biomasse zu Partikeln reduziert wird;
- b) Platzieren der Partikel auf einem Partikelfiltergitter, so dass die Partikel (P1), die kleiner als eine vorbestimmte Grösse von 5 mm sind, durch das Gitter durchlaufen und aus der ersten mechanischen Grössenreduktionsvorrichtung (1) herausgeführt werden;
- c) Lassen der Partikel (P1), die kleiner als die vorbestimmte Grösse sind, durch eine erste Partikelklassifizierungsvorrichtung (2) durchlaufen, um zu ermöglichen, dass die Partikel (P1), die kleiner als die vorbestimmte Grösse sind, entsprechend der Grösse in eine Fraktion (f1) von grobteiligen Partikeln und eine Fraktion (f2) von feinteiligen Partikeln getrennt zu werden, wobei in der Fraktion (f1) von grobteiligen Partikeln die Partikel grösser als 600 µm in Grösse sind, während in der Fraktion (f2) von feinteiligen Partikeln die Partikel kleiner als 600 µm sind, wobei die erste Klassifizierungsvorrichtung (2) ein Wirbelluftklassierer oder ein Zentrifugalsieb ist.

2. Verfahren nach Anspruch 1, wobei der Schritt b) des Durchlaufens der Partikel durch das Gitter von einem Schritt f) des Durchlaufens der Partikel (P1) gefolgt wird, die kleiner als die vorbestimmte Grösse sind und aus der ersten mechanischen Grössenreduktionsvorrichtung (1) oder aus dem Gitter durch mindestens eine zweite mechanische Grössenreduktionsvorrichtung (3) herausgeführt werden, um Partikel (P2,fi) zu erhalten, die kleiner sind als die aus der ersten mechanischen Grössenreduktionsvorrichtung (1) herausgeführten Partikel (P1) sind.

3. Verfahren nach Anspruch 2, wobei der Schritt f) des Durchlaufens der Partikel (P1), die kleiner als die vorbestimmte Grösse sind, durch die zweite mechanische Grössenreduktionsvorrichtung (3) durchgeführt wird, bevor die Partikel (P1), die kleiner als die vorbestimmte Grösse sind, in die erste Partikelklassifizierungsvorrichtung (2) zugeführt werden.

4. Verfahren nach einem der vorhergehenden Ansprüche 2 oder 3, umfassend einen Schritt des Durchlaufens der Fraktion (f1) von grobteiligen Partikeln, die aus der ersten Klassifizierungsvorrichtung (2) durch die zweite mechanische Grössenreduktionsvorrichtung (3) herausgeführt werden, um eine feinerteilige Fraktion (fi) zu erhalten.

5. Verfahren nach Anspruch 4, umfassend einen Schritt des Durchlaufens der feinerteiligen Partikel (fi) durch die erste Klassifizierungsvorrichtung, wo sie sich mit den aus der ersten mechanischen Grössenreduktionsvorrichtung (1) herausgeführten Partikeln (P1) mischen.

6. Verfahren nach einem der vorhergehenden Ansprüche, umfassend mindestens einen Schritt (e1, e2, e3), wobei die Fraktion (f2) von feinteiligen Partikeln durch mindestens eine zweite Partikelklassifizierungsvorrichtung (3a, 3b, 3c) durchlaufen gelassen wird, um der Fraktion (f2) von feinteiligen Partikeln zu ermöglichen, in zwei oder mehrere superfeine Fraktionen (f5,f5',f5',f7,f7',f7'', f8,f8',f8",f9,f10) getrennt zu werden, wobei jede davon Partikel einer vorbestimmten superfeinen Grösse aufweist.

7. Verfahren nach einem der vorhergehenden Ansprüche 2-5, umfassend mindestens einen Schritt (h), wobei die Fraktion (f2) von feinteiligen Partikeln durch mindestens eine dritte mechanische Partikelgrössenreduktionsvorrichtung (4) durchlaufen gelassen wird, um eine dritte Reduktion der Partikelgrösse der Fraktion (f2) auszuführen.

8. Verfahren nach Anspruch 7, wobei der mindestens eine Schritt (h) dem mindestens einen Schritt (e1) vorangeht, wobei die Fraktion (f2) von feinteiligen Partikeln durch die zweite Partikelklassifizierungsvorrichtung (3a, 3b, 3c) durchlaufen gelassen wird.

9. Verfahren nach einem der vorhergehenden Ansprüche 2-5, 7 oder 8, umfassend mindestens einen Schritt (i,e1;l,e2), wobei die Fraktion (f2) von feinteiligen Partikeln durch mindestens eine vierte Grössenreduktionsvorrichtung (5, 6) und mindestens eine nachfolgende dritte Partikelklassifizierungsvorrichtung (3b, 3c) durchlaufen gelassen wird, um zu ermöglichen, dass die Partikelgrösse allmählich reduziert wird und dass die Fraktion (f2) von feinteiligen Partikeln in zwei oder mehrere superfeine Fraktionen (f5'', f7", f8") dimensional getrennt wird.

10. Verfahren nach einem der vorhergehenden Ansprüche 6 oder 9, wobei in den superfeinen Fraktionen (f5,f5',f5",f7,f7',f7",f8,f8',f8",f9,f10) der Partikel mit einer vorbestimmten superfeinen Grösse die Partikel etwa jeweils die folgende Grösse aufweisen: 350 µm, 200 µm, 90 µm und kleiner als 90 µm.

11. Verfahren nach einem der vorhergehenden Ansprüche 6, 8 oder 9, wobei die weiteren Klassifizierungsvorrichtungen (3a, 3b, 3c) Wirbelluftklassierer oder Zentrifugalsiebe sind.

12. Verfahren nach einem der vorhergehenden Ansprüche 2-5 oder 7-9, wobei die zweite mechanische Grössenreduktionsvorrichtung (3) eine Zentrifugalprallmühle ist.

13. Verfahren nach einem der vorhergehenden Ansprüche 2-5, 7-9 oder 12, wobei die zweite mechanische Grössenreduktionsvorrichtung (3) in die erste Klassifizierungsvorrichtung (2) eingebaut ist oder umgekehrt.

14. Verfahren nach einem der vorhergehenden Ansprüche 2-5, 7-9, 12 oder 13, wobei die zweite mechanische Grössenreduktionsvorrichtung (3) eine Schleifvorrichtung oder eine Walzenmühle ist.

15. Verfahren zur Gewinnung von Biokraftstoffen aus Biomassen, umfassend die Schritte:
- Behandeln der Biomasse unter Verwendung des Vorbehandlungsverfahrens nach einem der vorhergehenden Ansprüche;
- Unterwerfen der aus dem Vorbehandlungsverfahren nach den vorhergehenden Ansprüchen erhaltenen Partikel zur biochemischen Konvertierung, um Biokraftstoff zu erhalten.

## Revendications

1. Procédé de prétraitement de biomasses pour permettre leur conversion ultérieure en biocarburant, **caractérisé en ce qu'**il comprend les étapes suivantes :
- a) faire passer les biomasses à travers un premier dispositif mécanique de réduction des dimensions (1) formé par un broyeur à couteaux ou un broyeur à marteaux pour permettre aux biomasses d'être réduites en particules ;
- b) placer les particules sur une grille de filtrage de particules de sorte que des particules (P1) inférieures à une dimension prédéfinie de 5 mm passent à travers la grille et ressortent du premier dispositif mécanique de réduction des dimensions (1) ;
- c) faire passer les particules (P1) étant plus petites que la dimension prédéfinie à travers un premier dispositif de classification de particules (2) pour permettre aux particules (P1) étant plus petites que la dimension prédéfinie d'être divisées selon la dimension en une fraction (f1) de particules de dimension grossière et une fraction (f2) de particules de dimensions fines, dans lequel dans la fraction (f1) de particules de dimensions grossières, lesdites particules ont une dimension supérieure à 600 µm, tandis que dans la fraction (f2) des particules de dimensions fines, lesdites particules ont une dimension inférieure à 600 µm, ledit premier dispositif de classification (2) étant un classificateur d'air à pulsion ou un tamis centrifuge.

2. Procédé selon la revendication 1, dans lequel l'étape b) de passage des particules à travers la grille est suivie d'une étape f) de passage des particules (P1) ayant une dimension inférieure à la dimension prédéfinie et qui ressortent du premier dispositif mécanique de réduction des dimensions (1), ou de la grille, à travers au moins un second dispositif mécanique de réduction des dimensions (3) pour obtenir des particules (P2, fi) ayant une dimension inférieure aux particules (P1) sortant du premier dispositif mécanique de réduction des dimensions (1).

3. Procédé selon la revendication 2, dans lequel l'étape f) de passage des particules (P1), ayant une dimension inférieure à la dimension prédéfinie, à travers le second dispositif mécanique de réduction des dimensions (3), est réalisée avant d'alimenter les particules (P1) ayant une dimension inférieure à la dimension prédéfinie dans le premier dispositif de classification des particules (2).

4. Procédé selon l'une quelconque des revendications précédentes 2 ou 3, comprenant une étape de passage de la fraction (f1) de particules de dimensions grossières sortant du premier dispositif de classification (2) à travers le second dispositif mécanique de réduction des dimensions (3) pour obtenir une fraction (fi) de dimensions plus fines.

5. Procédé selon la revendication 4, comprenant une étape de passage des particules de dimensions plus fines (fi) à travers le premier dispositif de classification où elles se mélangent avec les particules (P1) sortant du premier dispositif mécanique de réduction des dimensions (1).

6. Procédé selon l'une quelconque des revendications précédentes, comprenant au moins une étape (e1, e2, e3) consistant à faire passer la fraction (f2) de particules de dimensions fines à travers au moins un second dispositif de classification de particules (3a, 3b, 3c) pour permettre à la fraction (f2) de particules de dimensions fines d'être divisée selon leur dimension en deux ou plusieurs fractions superfines (f5, f5', f5'', f7, f7', f7'', f8, f8', f8'', f9, f10), chacune comportant des particules d'une dimension superfine prédéfinie.

7. Procédé selon l'une quelconque des revendication précédentes de 2 à 5, comprenant au moins une étape (h) de passage de la fraction (f2) de particules de dimensions fines à travers au moins un troisième dispositif mécanique de réduction des dimensions des particules (4) afin d'effectuer une troisième réduction de la dimension des particules de la fraction (f2).

8. Procédé selon la revendication 7, dans lequel l'au moins une étape (h) précède l'au moins une étape (e1) de passage de la fraction (f2) de particules de dimensions fines à travers le second dispositif de classification de particules (3a, 3b, 3c).

9. Procédé selon l'une quelconque des revendications précédentes de 2 à 5, 7 ou 8, comprenant au moins une étape (i, e1 ; 1, e2) de passage de la fraction (f2) de particules de dimensions fines à travers au moins un quatrième dispositif de réduction des dimensions (5, 6) et au moins un troisième dispositif de classification des particules (3b, 3c) ultérieur pour permettre à la dimension des particules d'être graduellement réduite et pour permettre à la fraction (f2) de particules de dimensions fines d'être divisée selon la dimension en deux ou plusieurs fractions superfines (f5'', f7'', f8").

10. Procédé selon l'une quelconque des revendications précédentes 6 ou 9, dans lequel dans les fractions superfines (f5, f5', f5", f7, f7', f7", f8, f8', f8", f9, f10) de particules de dimensions superfines prédéfinies, lesdites particules possèdent approximativement les dimensions suivantes, respectivement : 350 µm, 200 µm, 90 µm et inférieures à 90 µm.

11. Procédé selon l'une quelconque des revendications précédentes 6, 8 ou 9, dans lequel les dispositifs de classification (3a, 3b, 3c) supplémentaires sont des classificateurs d'air à pulsion ou des tamis centrifuges.

12. Procédé selon l'une quelconque des revendications précédentes de 2 à 5 ou de 7 à 9, dans lequel le second dispositif mécanique de réduction des dimensions (3) est un broyeur à percussion centrifuge.

13. Procédé selon l'une quelconque des revendications précédentes de 2 à 5, de 7 à 9 ou 12, dans lequel le second dispositif mécanique de réduction des dimensions (3) est construit dans le premier dispositif de classification (2) ou vice versa.

14. Procédé selon l'une quelconque des revendications précédentes de 2 à 5, de 7 à 9, ou 13, dans lequel le second dispositif mécanique de réduction des dimensions (3) est un dispositif de broyage ou un broyeur à cylindres.

15. Procédé servant à obtenir du biocarburant à partir de biomasses, comprenant les étapes de :
- traiter les biomasses en utilisant le procédé de prétraitement selon l'une quelconque des revendications précédentes ;
- soumettre les particules obtenues à partir du procédé de prétraitement selon les revendications précédentes à une conversion biochimique pour obtenir du biocarburant.
